# NEW EUROPEAN PATENT SPECIFICATION

(11) **EP 0 736 771 B2**
(45) Date of publication and mention of the opposition decision: **02.11.2005**
(45) Mention of the grant of the patent: 18.03.1998
(21) Application number: 95830238.2
(22) Date of filing: 07.06.1995
(51) Int. Cl.: G01N 33/76, G01N 33/558, G01N 33/543, A61B 5/00

(54) **Diagnostic method and test assembly for accurately and quickly detecting a clinical marker for diagnosing woman menopause**
Diagnostische Methode/Testvorrichtung zur exakten und schnellen Detektion eines klinischen Markers zur Diagnose der Menopause
Méthode et dispositif diagnostique pour la détection précise et rapide d'un marqueur clinique pour diagnostiquer la ménopause

(30) Priority: 06.04.1995 IT MI950702
(43) Date of publication of application: 09.10.1996
(73) Proprietor: OPPORTUNITY S.r.l., 20146 Milano (IT)
(72) Inventor: Bartoli, Ivan, I-20146 - Milano (IT)
(74) Representative: Modiano, Micaela Nadia

(56) References cited:
- EP-A- 0 238 353
- EP-A- 0 349 215
- WO-A-92/03738
- US-A- 4 762 783
- ANNALS OF CLINICAL BIOCHEMISTRY, vol. 17, no. 1, January 1980, LONDON, pages 38-44, XP000195716 LOVESEY: "A rapid solid-phase radioimmunoassay for human plasma follicle-stimulating hormone"
- Catalogue/manual of Syntron's "QuickPAcII One Step" no. 11820-25, 20.03.95.
- Zakur et al., Gynecol. Endocrinol., 1:263-268 (1987)
- Chang et al., "Physiology of the Menopause" . Editors Lorraine et al.Springer Verlag. (no date of publication indicated)
- Schaub et al., "Evaluation eines FSH Urin-tests zur schnellen Diagnostik der menopausalen Ovarialinsuffizienz" CDG/1/10.04.1998, pp 1-14, Published 1998

## Description

The present invention relates to a diagnostic test method and assembly for accurately and quickly detecting a clinical marker for diagnosing woman menopause.

As is known, the term "menopause" usually indicates the stop of the menstruation, with which a lacking of fertility is associated, since the ovary function has been exhausted and no further ovulation occurs and in which, sometimes, occur a series of signs and symptoms which can negatively effect the woman.

Usually the menopause is preceded by a period having a duration which can vary from some months to two or three years, in which great menstrual unevennesses can be present.

In the pre-menopause period, the ovary activity, i.e. the ovulation, as well as the production of estrogens and progesterone and the like, will decrease and progressively exhaust.

Such a decrease and exhaustion of the ovary activity must be timely diagnosed.

The progressive reduction of the estrogen production is also associated with an alteration of the lipide and bone metabolism which cause woman to be easily subjected to osteoporosis and cardiovascular pathologies. Medical treatments, on the other hand, can prevent these pathologies from occurring, but they must be timely started upon a diagnosis of menopause and exhaustion of the ovary function.

The diagnosis of menopause is based on an evidence of a stop of the menstruation, because of an exhaustion of the ovary activity.

Such a diagnosis is based on an appearance of signs and symptoms, such as vasomotor symptoms, i.e. bursts of heat occurring in a very large number of women, neurovegetative symptoms, such as tachycardia, paresthesia, dizziness, insomnia and the like, as well as psychosomatic symptoms, such as fatiguing, irritability, anxiety and the like, in addition to local genital symptoms such as vaginal dryness, dispareunia and the like.

The most evident endocrine modification consists of a persistent increase of the follicle stimulating hormone, also known as "FSH".

Contemporaneously the estrogens decrease and the LH, that is the luteinizing hormone increases.

Generally, the main diagnostic marker is the FSH, which has a comparatively high value even in menopause and, accordingly, allows to timely detect a reduction and exhaustion of the ovaric function.

At present, in order to properly detect the FSH, the latter is metered into the blood by immunoenzimatic methods or radioimmunologic methods on serum, by using specifically designed instruments (see, for example Lovesey Annals of Clinical Biochemistry, 17(1), 1980 pages 38-44) . The metering, which can be carried out exclusively by specialized medical personnel, involves a blood take-up operation and a hormone determination at an analysis laboratory.

Such a method involves a comparatively high cost and, mainly, a great discomfort for the patient.

A pre-ovulation test device is known and commercially available from Syntron Bioresearch Inc. under the trade name quik strip one step FSH test. This test has a sensitivity of 25 m/U/ml and is a urine-based test. The test has been commercially available at least since 1994.

### SUMMARY OF THE INVENTION

Accordingly, the aim of the present invention is to overcome the above mentioned problem, by providing a diagnostic test method and assembly for accurately and quickly detecting a clinical marker for diagnosing woman menopause, which allows the menopause test to be performed at home without requiring invasive blood collecting methods and affording the possibility of frequently repeating the test without any discomfort for the patient, and providing a result truly indicative of a menopause condition.

Another object of the present invention is to provide such a diagnostic test assembly which is very reliable and safe in operation.

Yet another object of the present invention is to provide such a diagnostic test assembly which is very competitive from a mere economic standpoint.

According to one aspect of the present invention, the above mentioned aim and objects, as well as yet other objects, which will become more apparent hereinafter, are achieved by a diagnostic test method for accurately and quickly detecting a clinical marker for diagnosing woman menopause according to claim 1 and by a diagnostic test assembly according to claim 2.

### BRIEF DESCRIPTION OF THE DRAWINGS

Further characteristics and advantages will become more apparent hereinafter from the following detailed disclosure of a preferred, though not exclusive, embodiment of a diagnostic test method and assembly for accurately and quickly detecting a clinical marker for diagnosing woman menopause, which is illustrated, by way of an indicative, but not limitative, example, in the figures of the accompanying drawings, where:

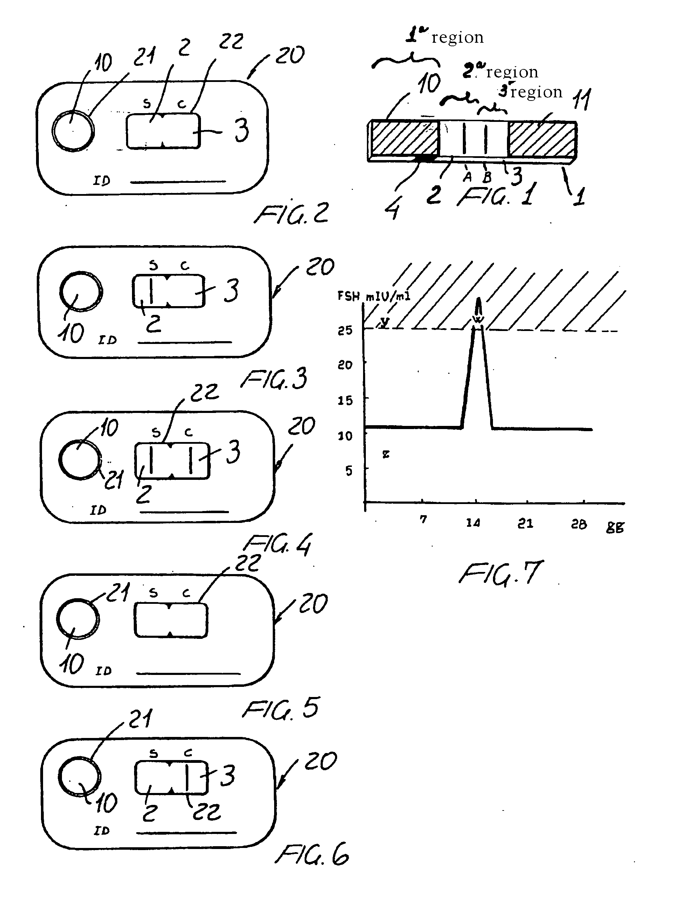

Figure 1 is a schematic view illustrating a strip thereon are arranged the chemical reagents;
Figure 2 illustrates the strip arranged in an user envelope;
Figure 3 is a schematic view illustrating the starting step of a test providing a positive result;
Figure 4 illustrates the end step of a test providing a positive result;
Figure 5 illustrates the starting step of a test providing a negative result;
Figure 6 illustrates the end step of a test providing a negative result;
   and
Figure 7 schematically illustrates the evolvement of the FSH hormone in the menstrual cycle.

### DESCRIPTION OF THE PREFERRED EMBODIMENT

With reference to the number references of the above mentioned figures, the diagnostic test assembly for accurately and quickly detecting a clinical marker for diagnosing woman menopause, according to the present invention, comprises a supporting strip 1, on which is provided a second region 2, where has been deposited and adhered in a dry form an anti-FSH polyclonal antibody, obtained from the rat, and which is commercially available.

The deposition can be performed either manually or automatically and is usually carried out at about 2.5 cm from the left end portion of the strip.

The anti-FSH antibody, which is colourless, is allowed to dry at room temperature and has an use concentration corresponding to the detection and responsibility requirements of the test.

The selection of the specific anti-FSH polyclonal antibody derives from the fact that it is necessary to perform a natural immunological reaction, under controlled conditions, between said antibody and the FSH antigen, that is the follicle stimulating hormone, which must be detected and which is considered to be present in a menopause woman urine at a detectable concentration, indicative of the menopause condition.

By the same deposition method, a third region 3 is made adjoining the second region 2, which second region 3 is arranged at about 3.5 cm from the left end portion of the strip, by causing an anti-immunoglobuline anti-antibody, in dry form, to adhere to the strip, which is also of a commercially available type.

Adjoining the region 2, at the portion thereof opposite to the region 3, there is formed a first region 4, having an anti-FSH monoclonal antibody, which is obtained by means of an immunization of a cavy animal, and also of a commercially available type.

On the left end portion of the supporting strip 1 there is moreover applied an imbibition layer 10, which is advantageously constituted by an absorption paper, bound on the cellulose acetate strip by means of natural organic glues; moreover, the application or imbibition layer 10 is superimposed on the first region (4), where is provided the anti-FSH monoclonal antibody.

The anti-FSH monoclonal antibody, in particular, is chemically bound by affinity with colloidal gold, for the intended specific applications, and is adapted to bind with the anti-immunoglobuline anti-antibody, as it will become more apparent hereinafter.

Finally, an absorption layer 11 is provided on the right end portion of the supporting strip 1 adjoining the region 3, at the portion thereof opposite to region 2, which layer 11 does not have any operating activity, but provides, in actual practice a stop layer against the absorption of the reaction liquid excess.

Advantageously, the thus prepared strip 1 is packaged within an envelope 20, having a circular hole 21 superimposed on the imbibition layer 10 and a double window 22, arranged at the second and third regions, and being provided, at the two regions, with indicating elements S and C which respectively correspond to a positive response and to a control.

By the above disclosed test assembly it is possible to easily perform the subject menopause test method, said method comprising, starting from a woman urine, the deposition of 5-7 drops at the window 21, so as to detect a possible presence of the hormone in the examined sample, at a concentration equal to or greater than 20 ml.U./ml.

The reaction, migration and colour fixation time, being indicative of the response, will be of about 8 minutes.

The urine will wet the layer 10 and will actuate, by a capillarity effect, the anti-FSH/colloidal gold assembly and FSH antigen.

As the migrating assembly will arrive at the region 2 having the anti-FSH polyclonal antibody, a second antigen-antibody reaction will occur which will be specific for the availability of three antihygienic places.

The "anchored" antibody will stop the assembly and the deposition will be made visible by a pink band, indicative of a positive result.

The migrating material, i.e. the complexed excess urine, will continue to displace by capillarity toward the right end portion of the strip 1, so as to safely react also with the anti-antibody material provided on the third region.

The latter reaction will always occur since it is independent from the presence of the FSH hormone in the urine.

Because of this characteristic and simple reaction between antibody and anti-antibody, which will be naturally mediated or driven, this further second "anchoring" will provide a second pink band which, in actual practice, will constitute a test control element.

In the case in which no FSH hormone is present in the urine, the urine which will have wet by capillarity the first region, will cause a driving or displacement of the antibody anti-FSH/colloidal gold assembly not bound to any antigens.

As the migrating assembly will arrive at the second region, it will be not captured, thereby no reaction will occur, and the second region will not have any coloured strip, thereby indicating a negative result.

The remaining sequence of the reaction occurs as previously disclosed, by forming a pink band at the third region.

According to the invention, the test method must be repeated after about 15 days, since a positive result of the test merely indicates that, for the time, is present a concentration of the FSH hormone different from the normal concentration.

Since, however, the menstrual cycle has a duration of several days, which generally corresponds to the ovulation period, in which the FSH hormone increases to values of about 25 ml.U./ml, it is necessary to verify that the FSH hormone is constantly present at greater concentrations, whereby only a persistence of high levels will indicate an exhaustion of the ovulation function and a menopause condition.

From experimental tests, it has been found that a double positive result, obtained from tests performed at a distance of about 15 days from one another, provides an absolute warranty of the presence of the FSH hormone, thereby the user will be sure of her menopause physiologic condition.

Moreover, in the case in which one of the two tests provides a negative result, this is indicative of the fact that the woman is not in a menopause condition.

From the above disclosure it should be apparent that the invention fully achieves the intended aim and objects.

In particular, it is stressed the great simplicity of making of the test according to the present invention, which can be performed at home, without any discomfort and at a very reduced cost.

In practicing the invention, the used materials, provided that they are compatible to the intended use, as well as the contingent size and shapes, can be any, depending on to requirements.

## Claims

1. A method for accurately and quickly detecting a clinical marker for diagnosing woman menopause, said method comprising the steps of
a) providing a diagnostic test assembly including
i) a supporting strip packaged within an envelope (20) having a circular hole (21), said supporting strip supporting thereon;
ii) a first region (4), where an anti-FSH monoclonal antibody is located;
iii) a second region (2) adjoining said first region (4) where an anti-FSH polyclonal antibody is located;
iv) a third region (3) adjoining said second region (2) where an antiimmunoglobulin IgG antibody is located, which can bind with said monoclonal anti-FSH antibody;
v) an imbibition layer (10) superimposed on said first region (4);
vi) an absorption layer (11) on an end of said strip (1) adjoining said third region (3);
vii) said envelope (20) further having a rectangular window (22) arranged at said second (2) and third (3) regions;
b) depositing drops of a woman's urine through said window (21) to cause said urine to migrate by capillarity from said first region (4) through said second (2) and third (3) region toward said end of said strip (1) adjoining said third region (3) and to react therewith so as to detect a possible presence of the FSH hormone at a concentration equal to or greater than 20mlU/ml,
**characterized in that** said method comprises the further step of
c) if in said step b) an amount of said FSH hormone equal to or greater than 20 mlU/ml has been detected as a first positive result, repeating, fifteen days later, said steps a) and b) so as to obtain a second positive result providing, in combination with said first positive result, an accurate indication of a true menopause condition.

2. A diagnostic test assembly for accurately and quickly diagnosing menopause in women by detecting FSH in a urine sample, said diagnostic test assembly comprising;
i) a supporting strip packaged within an envelope (20) having a circular hole (21) superimposed on an imbibition layer (10), which layer is capable of being method with said urine sample , said supporting strip supporting thereon;
ii) a first region (4), where an anti-FSH monoclonal antibody is located;
iii) a second region (2) adjoining said first region (4) where an anti-FSH polyclonal antibody is located;
iv) a third region (3) adjoining said second region (2) where an antiimmunoglobulin IgG antibody is located, which can bind with said monoclonal anti-FSH antibody;
v) an imbibition layer (10) superimposed on said first region (4);
vi) said absorption layer (11) on an end of said strip (1) adjoining said third region (3);
vii) said envelope (20) further having a rectangular window (22) arranged at said second (2) and third (3) regions,
wherein the assembly defects a possible presence of the FSH hormone in said urine sample at a concentration equal to or greater than 20mlU/ml.

3. A diagnostic test assembly according to claim 2, **characterized in that** said anti-FSH polyclonal antibody is deposited on and adhered in a dry form to the supporting strip (1).

4. A diagnostic test assembly according to claims 2 and 3, **characterized in that** said anti-FSH monoclonal antibody is provided on an absorbing paper layer which has been dry impregnated and underlies said imbibition layer (10).

5. A diagnostic test assembly according to one or more of the preceding claims, **characterized in that** said imbibition layer (10) comprises absorbing paper bound to said supporting strip.

6. A diagnostic test assembly according to one or more of the preceding claims, **characterized in that** said absorbing layer (11) comprises and absorbs paper exclusively operating for absorbing the reaction liquid excess.

## Patentansprüche

1. Ein Verfahren, um auf genaue und rasche Weise einen klinischen Marker für die Diagnose der Wechseljahre von Frauen zu erkennen, umfassend die Schritte
a) Bereitstellen einer diagnostischen Testvorrichtung einschließlich
i) eines in einer Hülle (20) verpackten Tragestreifens mit einer kreisförmigen Bohrung (21), wobei auf diesem Tragestreifen gelagert sind:
ii) ein erster Bereich (4), in dem sich ein anti-FSH monoklonaler Antikörper befindet;
iii) ein an diesen ersten Bereich (4) angrenzender zweiter Bereich (2), in dem sich ein anti-FSH polyklonaler Antikörper befindet;
iv) ein an diesen zweiten Bereich (2) angrenzender dritter Bereich (3), in dem sich ein antiimmunoglobulin-IgG-Antikörper befindet, der diesen monoklonalen anti-FSH-Antikörper-Bereich binden kann;
v) eine auf diesem ersten Bereich (4) liegende Tränkungsschicht (10); vi) eine Absorptionsschicht (11), die an einem Ende dieses Streifens (1) an diesen dritten Bereich (3) angrenzt;
vii) wobei diese Hülle (20) zudem ein in diesem zweiten (2) und dritten (3) Bereich angeordnetes rechteckiges Fenster (22) besitzt;
b) Abscheiden von Frauenurintropfen durch diese Bohrung (21), damit dieses Urin mittels Kapillarwirkung von diesem ersten Bereich (4) über diesen zweiten (2) und dritten (3) Bereich zu diesem Ende dieses an diesen dritten Bereich (3) angrenzenden Streifens (1) wandert und mit diesem reagiert, um so ein mögliches Vorliegen des FSH-Hormons in einer Konzentration, die gleich oder größer als 20mlU/ml ist, zu erkennen,
**dadurch gekennzeichnet, dass** dieses Verfahren die weiteren Schritte umfasst:
c) wenn in diesem Schritt b) eine Menge dieses FSH-Hormons gleich oder größer als 20mlU/ml als ein erstes positives Ergebnis erkannt worden ist, Wiederholung fünfzehn Tage später dieser Schritte a) und b), um so ein zweites positives Ergebnis zu erzielen und in Verbindung mit diesem ersten positiven Ergebnis einen genauen Hinweis auf einen wirklichen Wechseljahrezustand liefert.

2. Eine diagnostische Testvorrichtung, um auf genaue und rasche Weise die Wechseljahre bei Frauen zu diagnostizieren, indem FSH in einer Urinprobe erkannt wird, wobei diese Testvorrichtung umfasst:
i) einen in einer Hülle (20) verpackten Tragestreifen mit einer auf einer Tränkungsschicht (10) liegenden kreisförmigen Bohrung (21), wobei diese Schicht geeignet ist, um mit dieser Urinprobe befeuchtet zu werden, wobei auf diesem Tragestreifen gelagert sind:
ii) ein erster Bereich (4), in dem ein sich anti-FSH monoklonaler Antikörper befindet;
iii) ein an den ersten Bereich (4) angrenzender zweiter Bereich (2), in dem sich ein anti-FSH monoklonaler Antikörper befindet;
iv) ein an diesen zweiten Bereich (2) angrenzender dritter Bereich (3), in dem sich ein antiimmunoglobulin-IgG-Antikörper befindet, der diesen monoklonalen anti-FSH-Antikörper-Bereich binden kann;
v) eine auf diesem ersten Bereich (4) liegende Tränkungsschicht (10);
vi) eine Absorptionsschicht (11), die an einem Ende dieses Streifens (1) an diesen dritten Bereich (3) angrenzt;
vii) wobei diese Hülle (20) zudem ein an diesem zweiten (2) und dritten (3) Bereich angeordnetes rechteckiges Fenster (22) besitzt,
in der die Vorrichtung ein mögliches Vorliegen des FSH-Hormons in dieser Urinprobe in einer Konzentration, die gleich oder größer als 20mlU/ml ist, erkennt.

3. Eine diagnostische Testvorrichtung nach Anspruch 2, **dadurch gekennzeichnet, dass** dieser anti-FSH polyklonaler Antikörper in trockener Form auf dem Tragestreifen (1) abgeschieden und daran haften lassen wird.

4. Eine diagnostische Testvorrichtung nach den Ansprüchen 2 und 3, **dadurch gekennzeichnet, dass** dieser anti-FSH monoklonaler Antikörper auf einer Schicht aus absorbierendem Papier bereitgestellt ist, die trocken imprägniert ist und unter dieser Tränkungsschicht (10) liegt.

5. Eine diagnostische Testvorrichtung nach einem oder mehreren der vorhergehenden Ansprüche, **dadurch gekennzeichnet, dass** diese Tränkungsschicht (10) absorbierendes Papier umfasst, das mit diesem Tragestreifen verbunden ist.

6. Eine diagnostische Testvorrichtung nach einem oder mehreren der vorhergehenden Ansprüche, **dadurch gekennzeichnet, dass** diese Absorptionsschicht (11) Papier umfasst und absorbiert, das allein zur Aufnahme der überschüssigen Reaktionsflüssigkeit dient.

## Revendications

1. Méthode pour la détection fiable et rapide d'un marqueur clinique pour diagnostiquer la ménopause chez la femme, ladite méthode comprenant les étapes consistant à
a) fournir un dispositif d'essai diagnostique qui comprend
i) une bandelette de support conditionnée dans une enveloppe (20) présentant un trou circulaire (21), ladite bandelette de support supportant;
ii) une première zone (4), dans laquelle est placé un anticorps monoclonal anti-FSH;
iii) une deuxième zone (2) avoisinant ladite première zone (4), dans laquelle est placé un anticorps polyclonal anti-FSH;
iv) une troisième zone (3) avoisinant ladite deuxième zone (2), dans laquelle est placé un anticorps anti-immunoglobuline IgG capable de lier ledit anticorps monoclonal anti-FSH;
v) une couche d'imbibition (10) surimposée à ladite première zone (4);
vi) une couche d'absorption (11) sur une extrémité de ladite bandelette (1) avoisinant ladite troisième zone (3);
vii) ladite enveloppe (20) présentant en outre une fenêtre rectangulaire (22) agencée dans lesdites deuxième (2) et troisième (3) zones;
b) déposer des gouttes d'urine d'une femme dans ledit trou (21) pour provoquer la migration par capillarité de ladite urine depuis la première zone (4) à travers lesdites deuxième (2) et troisième (3) zones vers ladite extrémité de la dite bande (1) avoisinant ladite troisième zone (3) et la réaction avec celle-ci pour détecter la présence éventuelle de l'hormone FSH à une concentration égale ou supérieure à 20mlU/ml,
**caractérisée en ce que** ladite méthode comprend l'étape supplémentaire consistant à
c) répéter, quinze jours plus tard, les étapes a) et b) si durant ladite étape b) une valeur de ladite hormone FSH égale ou supérieure à 20mlU/ml a été détectée comme premier résultat positif, afin d'obtenir un deuxième résultat positif qui fourni, en combinaison avec le premier résultat positif, une indication fiable d'une condition réelle de ménopause.

2. Dispositif d'essai diagnostique pour le diagnostic fiable et rapide de la ménopause par détection de FSH dans un échantillon d'urine, ledit dispositif d'essai diagnostique comprenant:
i) une bandelette de support conditionnée dans une enveloppe (20) présentant un trou circulaire (21) surimposé à une couche d'imbibition (10) pouvant être humidifiée par ledit échantillon d'urine, ladite bandelette de support supportant;
ii) une première zone (4), dans laquelle est placé un anticorps monoclonal anti-FSH;
iii) une deuxième zone (2) avoisinant ladite première zone (4), dans laquelle est placé un anticorps polyclonal anti-FSH;
iv) une troisième zone (3) avoisinant ladite deuxième (2), dans laquelle est placé un anticorps anti-immunoglobuline IgG capable de lier ledit anticorps monoclonal anti-FSH;
v) ladite couche d'imbibition (10) surimposée à ladite première zone (4);
vi) une couche d'absorption (11) sur une extrémité de ladite bandelette (1) avoisinant ladite troisième zone (3);
vii) ladite enveloppe (20) présentant en outre une fenêtre rectangulaire (22) agencée dans lesdites deuxième (2) et troisième (3) zones,
dans lequel le dispositif détecte la présence éventuelle de l'hormone dans un échantillon d'urine à une concentration égale ou supérieure à 20mlU/ml.

3. Dispositif d'essai diagnostique selon la revendication 2, **caractérisé en ce que** ledit anticorps polyclonal anti-FSH est déposé sur et fait adhéré à sec à ladite bandelette de support (1).

4. Dispositif d'essai diagnostique selon les revendications 2 et 3, **caractérisé en ce** ledit anticorps monoclonal anti-FSH est agencé sur une couche de papier absorbant qui a été imprégné à sec et qui est sous-jacent à ladite couche d'imbibition (10).

5. Dispositif d'essai diagnostique selon l'une ou plusieurs des revendications précédentes,
**caractérisé en ce** ladite couche d'imbibition (10) comprend du papier absorbant lié à ladite bandelette de support.

6. Dispositif d'essai diagnostique selon l'une ou plusieurs des revendications précédentes,
**caractérisé en ce** ladite couche d'absorption (11) comprend et absorbe du papier dont la seule tâche est d'absorber le liquide de réaction en excès.
